# EUROPÄISCHE PATENTANMELDUNG

(11) **EP 1 249 443 A2**
(43) Veröffentlichungstag der Anmeldung: **16.10.2002**
(21) Anmeldenummer: 02007273.2
(22) Anmeldetag: 02.04.2002
(51) Int. Cl.: C07C 67/10, C07C 69/007, C07C 69/88

(54) **Verfahren zur Herstellung von Hydroxybenzoesäurebenzylestern**

(30) Priorität: 12.04.2001 DE 10118308
(71) Anmelder: BAYER AG, 51368 Leverkusen (DE)
(72) Erfinder: Ooms, Pieter, Dr., 47800 Krefeld (DE); Schenke, Bernd-Ulrich, 46236 Bottrop (DE)

(57) **Zusammenfassung**

Hydroxybenzoesäurebenzylestern können durch Umsetzung von Benzylchlorid mit Hydroxybenzoesäuren hergestellt werden.

## Beschreibung

Die vorliegende Erfindung betrifft ein Verfahren zur Herstellung von Hydroxybenzoesäurebenzylestern durch Umsetzung von Benzylchlorid mit Hydroxybenzoesäure.

Benzylsalicylat wird als Stabilisator in Duftkompositionen und Sonnenschutzmitteln eingesetzt.

Benzylsalicylat und Verfahren zu seiner Herstellung sind bereits bekannt.

So wird in der EP-A 117 502 beispielsweise die Herstellung von Benzylsalicylat durch Veresterung von Salicylsäure oder Umesterung von Salicylsäureestern mit Benzylalkohol beschrieben.

Benzylsalicylat kann auch durch Umsetzung von Alkalisalicylaten mit Benzylchlorid gegebenenfalls in Gegenwart von Phasentransferreagenzien hergestellt werden (JP-A 63/218652, EP-A 534817). Nachteilig ist die Bildung von Salzen, die entsorgt werden müssen und somit die Wirtschaftlichkeit dieser Verfahren verringern.

Es bestand also die Aufgabe, ausgehend von Benzylchlorid ein Verfahren zur Herstellung von Hydroxybenzoesäurebenzylestern zu entwickeln, welches unter milden Reaktionsbedingungen durchführbar ist und kostengünstig zu guten Ausbeuten führt.

Es wurde ein Verfahren zur Herstellung von Hydroxybenzoesäurebenzylestern der Formel 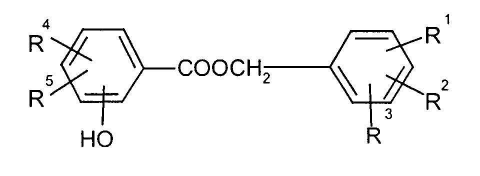 worin
- R¹ bis R⁵: gleich oder verschieden sind und für Wasserstoff, C₁-C₆-Alkyl, C₁-C₆-Alkoxy-, C₁-C₆-Halogenalkyl, C₁-C₆-Halogenalkoxy-, CN, CO(C₁-C₆-Alkyl), NO₂ oder Halogen stehen,
aus Benzylchloriden gefunden, das dadurch gekennzeichnet ist, dass Benzylchloride der Formel 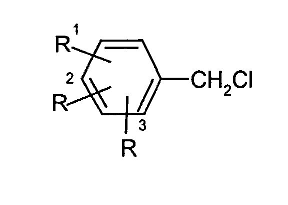 worin
- R¹, R² und R³: die oben genannte Bedeutung haben,
oder Gemische aus Benzylchloriden und Benzylalkoholen der Formel 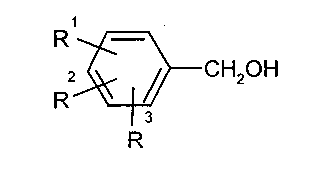 worin
- R¹, R² und R³: die oben genannte Bedeutung haben,
mit Hydroxybenzoesäuren der Formel 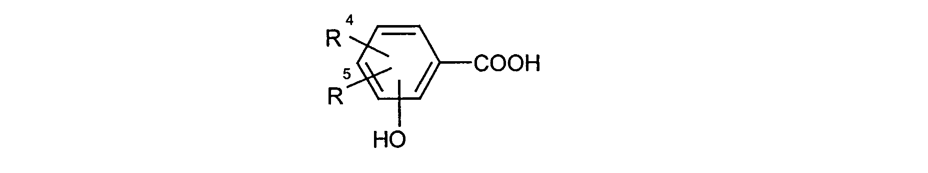 worin
R⁴ und R⁵ die oben genannte Bedeutung haben,
in Gegenwart einer oder mehrerer Amiden der Formel

R⁶CONR⁷R⁸

worin
- R⁶: Wasserstoff, C₁-C₆-Alkyl, C₁-C₆-Alkoxy-, Amino-, C₁-C₆-Alkylamino, C₁-C₆-Cycloalkylamino oder C₁-C₆-Dialkylamino bedeutet und
- R⁷ und R⁸: gleich oder verschieden sind und Wasserstoff, C₁-C₆-Alkyl, C₁-C₆-Cycloalkyl, C₁-C₆-Alkoxy-, C₁-C₁₀-Aralkyl oder C₁-C₁₂-Aryl bedeuten,
oder
- R⁶ und R⁷: gemeinsam eine Gruppe (CH₂)ₙ oder NR⁹(CH₂)ₙ bilden,
worin
- n: 2 bis 6 bedeutet,
umgesetzt werden.

Das erfindungsgemäße Verfahren lässt sich kostengünstig und unter milden Reaktionsbedingungen durchführen.

Die Reste R¹ bis R⁵ haben im allgemeinen die folgende Bedeutung: Alkyl bedeutet im allgemeinen ein geradkettiger oder verzweigter Kohlenwasserstoffrest mit 1 bis 6, bevorzugt 1 bis 4, im besondere bevorzugt 1 oder 2 Kohlenstoffatomen. Beispielsweise seien Methyl, Ethyl, Propyl, iso-Propyl, Butyl, iso-Butyl, Pentyl, iso-Pentyl, Hexyl und iso-Hexyl genannt. Bevorzugt sind Methyl, Ethyl, Propyl, iso-Propyl, Butyl, Pentyl und Hexyl, im besondere bevorzugt sind Methyl und Ethyl.

Alkoxy bedeutet im allgemeinen ein geradkettiger oder verzweigter Alkoxyrest mit 1 bis 6, bevorzugt 1 bis 4, im besondere bevorzugt 1 oder 2 Kohlenstoffatomen. Beispielsweise seien Methoxy, Ethoxy, Propoxy, iso-Propoxy, Butoxy, iso-Butoxy, Pentoxy, iso-Pentoxy, Hexoxy und iso-Hexoxy genannt. Bevorzugt sind Methoxy, Ethoxy, Propoxy, iso-Propoxy, Butoxy, iso-Butoxy, Pentoxy und Hexoxy, im besondere bevorzugt sind Methoxy und Ethoxy.

Halogenalkyl bedeutet im allgemeinen ein geradkettiger oder verzweigter Kohlenwasserstoffrest mit 1 bis 6, bevorzugt 1 bis 4, im besondere bevorzugt 1 oder 2 Kohlenstoffatomen mit 1 bis 10, bevorzugt 1 bis 8, im besondere bevorzugt mit 1 bis 5 Halogenatomen. Beispielsweise seien Chlormethyl, Fluormethyl, Difluormethyl, Trifluormethyl, Fluorethyl, Fluorpropyl und Hexafluorbutyl genannt. Bevorzugt sind Fluormethyl, Difluormethyl, Trifluormethyl, Fluorethyl, Fluorpropyl und Hexafluorbutyl, im besondere bevorzugt sind Fluormethyl und Trifluormethyl.

Halogenalkoxy bedeutet im allgemeinen ein geradkettiger oder verzweigter Alkoxyrest mit 1 bis 6, bevorzugt 1 bis 4, im besondere bevorzugt 1 oder 2 Kohlenstoffatomen mit 1 bis 10, bevorzugt 1 bis 8, im besondere bevorzugt mit 1 bis 5 Halogenatomen. Beispielsweise seien Chlormethoxy, Fluormethoxy, Difluormethoxy, Trifluormethoxy, Fluorethoxy, Fluorpropoxy und Hexafluorbutoxy genannt. Bevorzugt sind Chlormethoxy, Fluormethoxy, Trifluormethoxy, Fluorethoxy, Fluorpropoxy und Hexafluorbutoxy, im besondere bevorzugt sind Fluormethoxy und Trifluormethoxy.

Halogen bedeutet im allgemeinen Fluor, Chlor, Brom und Jod, bevorzugt Fluor, Chlor und Brom, im besondere Fluor und Chlor.

Ganz besonders bevorzugte Substituenten für R¹ bis R⁵ sind Wasserstoff, Methyl, Trifluormethyl, Methoxy, Fluor oder Chlor.

Nach dem erfindungsgemäßen Verfahren können beispielsweise die folgenden Hydroxybenzoesäurebenzylestern hergestellt werden:
2-Hydroxybenzoesäurebenzylester (Salicylsäurebenzylester); 3-Hydroxybenzoesäurebenzylester, 4-Hydroxybenzoesäurebenzylester; 3-Chlor-2-hydroxybenzoesäurebenzylester, 4-Chlor-2-hydroxybenzoesäurebenzylester, 5-Chlor-2-hydroxybenzoesäurebenzylester; 6-Chlor-2-hydroxybenzoesäurebenzylester, 3-Brom-2-hydroxybenzoesäurebenzylester, 3-Fluor-2-hydroxybenzoesäurebenzylester, 4-Fluor-2-hydroxybenzoesäurebenzylester, 2-Fluor-3-hydroxybenzoesäurebenzylester, 2-Fluor-4-hydroxybenzoesäurebenzylester; 3-Fluor-2-hydroxybenzoesäurebenzylester, 2-Fluor-5-hydroxybenzoesäurebenzylester, 2-Fluor-6-hydroxybenzoesäurebenzylester, 2-Hydroxy-3-methylbenzoesäurebenzylester, 2-Hydroxy-4-methylbenzoesäurebenzylester, 3-Hydroxy-2-methylbenzoesäurebenzylester, 4-Hydroxy-2-methylbenzoesäurebenzylester, 2-Fluor-6-hydroxy-4-methoxybenzoesäurebenzylester, 3-Trifluormethyl-2-hydroxybenzoesäurebenzylester, 4-Trifluormethyl-2-hydroxybenzoesäurebenzylester, 2-Trifluormethyl-3-hydroxybenzoesäurebenzylester, 2-Fluorethyl-4-hydroxybenzoesäurebenzylester und 4-Fluorbutyl-2-hydroxybenzoesäurebenzylester.

Bei dem im erfindungsgemäßen Verfahren eingesetzten Benzylchlorid handelt es sich um unsubstituiertes oder ein substituiertes Benzylchlorid.

Besonders bevorzugt wird unsubstituiertes Benzylchlorid eingesetzt.

In das erfindungsgemäßen Verfahren können Benzylchlorid oder Benzylchlorid/ Benzylalkoholgemische, wie sie beispielsweise bei der Herstellung von Benzylalkohol aus Benzylchlorid anfallen, eingesetzt werden. Der Gehalt an Benzylchlorid in dem Gemisch kann 50 bis 100 %, bevorzugt 60 bis 95 %, besonders bevorzugt 70 bis 90 % sein.

Für das erfindungsgemäße Verfahren seien beispielsweise die folgenden Hydroxybenzoesäuren genannt:
2-Hydroxybenzoesäure (Salicylsäure), 3-Hydroxybenzoesäure, 4-Hydroxybenzoesäure, 3-Chlor-2-hydroxybenzoesäure, 4-Chlor-2-hydroxybenzoesäure, 5-Chlor-2-hydroxybenzoesäure, 6-Chlor-2-hydroxybenzoesäure, 3-Brom-2-hydroxybenzoesäure, 3-Fluor-2-hydroxybenzoesäure, 4-Fluor-2-hydroxybenzoesäure, 5-Fluor-2-hydroxybenzoesäure, 6-Fluor-2-hydroxybenzoesäure, 2-Fluor-3-hydroxybenzoesäure, 2-Fluor-4-hydroxybenzoesäure, 2-Fluor-5-hydroxybenzoesäure, 2-Fluor-6-hydroxybenzoesäure, 2-Hydroxy-3-methylbenzoesäure, 2-Hydroxy-4-methylbenzoesäure, 3-Hydroxy-2-methylbenzoesäure, 4-Hydroxy-2-methylbenzoesäure, 6-Hydroxy-2-fluor-4-methoxybenzoesäure, 2-Hydroxy-3-trifluormethyl-benzoesäure, 2-Hydroxy-4-trifluormethylbenzoesäure, 3-Hydroxy-2-trifluormethyl-benzoesäure, 4-Hydroxy-2-fluorethylbenzoesäure, 2-Hydroxy-4-fluorbutyl-benzoesäure, 2-Hydroxy-3-methoxybenzoesäure, 2-Hydroxy-4-nitrobenzoesäure, 3-Acetyl-2-hydroxybenzoesäure oder 4-Cyan-2-hydroxybenzoesäure.

Bevorzugt sind Hydroxybenzoesäuren mit 2 bis 30 C-Atomen, im besondere bevorzugt 2 bis 12 C-Atomen. Ganz besonders bevorzugt sind Salicylsäuren.

Das erfindungsgemäße Verfahren wird vorzugsweise unter Entfernung des gebildeten Wasserstoffchlorids durchgeführt. Geeignet ist die Entfernung des Chlorwasserstoffes durch Durchleiten eines inerten Gases wie beispielsweise Stickstoff.

Im erfindungsgemäßen Verfahren werden vorzugsweise 0,1 bis 50 Mol an Hydroxybenzoesäure, bevorzugt 0,5 bis 30 Mol, besonders bevorzugt 1 bis 20 Mol, bezogen auf 1 Mol Benzylchlorid eingesetzt.

Als Amide werden Verbindungen der Formel

R⁶CONR⁷R⁸

worin
- R⁶: Wasserstoff, C₁-C₆-Alkyl, C₁-C₁₀-Aralkyl, C₁-C₁₂-Aryl, C₁-C₆-Alkoxy-, Amino-, C₁-C₆-Alkylamino, C₁-C₆-Cycloalkylamino oder C₁-C₆-Dialkylamino bedeutet und
- R⁷ und R⁸: gleich oder verschieden sind und Wasserstoff, C₁-C₆-Alkyl, C₁-C₆-Cycloalkyl, C₁-C₁₀-Aralkyl oder C₁-C₁₂-Aryl bedeuten
oder
- R⁶ und R⁷: gemeinsam eine Gruppe (CH₂)ₙ oder NR⁹ (CH₂)ₙ bilden,
worin
- R⁹: Wasserstoff, C₁-C₆-Alkyl, C₁-C₆-Cycloalkyl, C₁-C₁₀-Aralkyl oder C₁-C₁₂-Aryl bedeutet
und
- n: 1 bis 5 bedeutet,
genannt.

Für die Durchführung des erfindungsgemäßen Verfahren geeignete Amide sind geradkettig oder verzweigt, acyclisch oder cyclisch.

Geeignet sind Amide, Alkylamide-, Dialkylamide-, Cycloalkylamide-, Arylalkylamide- oder Arylamide von Carbonsäuren mit 1 bis 30 C-Atomen und Harnstoffe, Mono-, Di-, Tri- oder Tetraalkylharnstoffe mit 1 bis 30 C-Atomen.

Bevorzugt sind Formamid, N-Methylformamid, N-Ethylformamid, Dimethylformamid, Acetamid, Methylacetamid, Harnstoff, Dimethylhamstoff oder Tetramethylharnstoff, im besondere bevorzugt sind Formamid, N-Methylformamid, Acetamid, Methylacetamid, Harnstoff, Dimethylharnstoff oder Tetramethylharnstoff.

Weiterhin geeignet sind cyclische Amide (Lactame) von Amino-, Alkylamino-, Arylamino-, Hydroxyamino-, Alkoxyamino-, Alkoxycarbonylamino- und Alkoxycarbonylalkylaminocarbonsäuren mit 1 bis 30 C-Atomen.

Als Alkyl geeignet sind geradkettige oder verzweigte, cyclische oder acyclische Alkyl-, Aryl-, Aralkyl-, Hydroxyalkyl-, Alkoxyalkyl- und Alkoxycarbonylresten mit 1 bis 30 C-Atomen, bevorzugt 1 bis 24 C-Atomen, im besondere bevorzugt 1 bis 20 C-Atomen, bevorzugt Alkyl-, Aralkyl-, Aryl-, Hydroxyalkyl- und Alkoxyalkyl-, besonders bevorzugt Alkyl-, Aralkyl- und Hydroxyalkylresten.

Als Lactame kommen vier-, fünf-, sechs-, sieben- und höhergliedrige cyclische Amide mit 1 bis 30 C-Atomen wie beispielsweise 2-Pyrrolidinone, 2-Piperidone und Caprolactame in Frage.

Weiterhin geeignet sind aromatische Lactame wie beispielsweise 2-Pyridone oder 1-Alkyl-2-pyridone.

Die zur Herstellung der erfindungsgemäßen Katalysatoren eingesetzten Lactame werden in der Regel durch Cyclisierung von β-, γ-, δ- oder ε-Aminocarbonsäuren oder -carbonsäureester mit 1 bis 30 C-Atomen gegebenenfalls in Gegenwart von Katalysatoren erhalten.

Geeignet sind Pyrrolidinon, N-Alkylpyrrolidinon, N-Cycloalkyl-2-pyrrolidinon, N-Hydroxyalkyl-2-pyrrolidinon, 2-Piperidon, N-Alkyl-2-piperidon, N-Cycloalkyl-2-piperidon, N-Aryl-2-piperidon, Caprolactam, N-Alkyl-2-caprolactam, N-Vinyl-2-caprolactam, N-Hydroxyalkyl-2-caprolactam, N-Aryl-2-caprolactam, N-Alkyl-2-pyridon, N-Cycloalkyl-2-pyridon, N-Hydroxyalkyl-2-pyridon und N-Aryl-2-pyridon.

Bevorzugte Lactame sind 2-Pyrrolidinon, N-Methyl-2-pyrrolidinon, N-Ethyl-2-pyrrolidinon, N-Propyl-2-pyrrolidinon, N-Butyl-2-pyrrolidinon, N-Pentyl-2-pyrrolidinon, N-Hexyl-2-pyrrolidinon, N-Heptyl-2-pyrrolidinon, N-Octyl-2-pyrrolidinon, N-Nonyl-2-pyrrolidinon, N-Decyl-2-pyrrolidinon, N-Undecyl-2-pyrrolidinon, N-Dodecyl-2-pyrrolidinon, N-Hexadecyl-2-pyrrolidinon, N-Heptadecyl-2-pyrrolidinon, N-Octadecyl-2-pyrrolidinon, N-Cyclopentyl-2-pyrrolidinon, N-Cyclohexyl-2-pyrrolidinon, N-Vinyl-2-pyrrolidinon, N-Hydroxyethyl-2-pyrrolidinon, N-Hydroxypropyl-2-pyrrolidinon, N-Hydroxybutyl-2-pyrrolidinon, N-Benzyl-2-pyrrolidinon, N-Phenyl-2-pyrrolidinon, N-Methyl-2-piperidon, N-Ethyl-2-piperidon, N-Propyl-2-piperidon, N-Propyl-2-piperidon, N-Cyclohexyl-2-piperidon, N-Hydroxyethyl-2-piperidon, N-Vinyl-2-piperidon, N-Benzyl-2-piperidon, N-Phenyl-2-piperidon, 2-Caprolactam, N-Methyl-2-caprolactam, N-Ethyl-2-caprolactam, N-Propyl-2-caprolactam, N-Butyl-2-caprolactam, N-Propyl-2-caprolactam, N-Cyclohexyl-2-caprolactam, N-Vinyl-2-caprolactam, N-Hydroxyethyl-2-caprolactam, N-Methyl-2-pyridon, N-Ethyl-2-pyridon, N-Propyl-2-pyridon, N-Cyclohexyl-2-pyridon, N-Vinyl-2-pyridon, N-Benzyl-2-pyridon, N-Phenyl-2-pyridon oder N-Hydroxyethyl-2-pyridon, im besondere bevorzugt sind 2-Pyrrolidinon, N-Methyl-2-pyrrolidinon, N-Ethyl-2-pyrrolidinon, N-Cyclohexyl-2-pyrrolidinon, 2-Caprolactam, N-Methyl-2-caprolactam und N-Ethyl-2-caprolactam.

Methoden zur Herstellung von Amiden sind allgemein gut bekannt und beispielsweise ausführlich beschrieben in "Römpp: Lexikon Chemie", 10. Auflage, Stuttgart/New York 1997, Band 1, S.153, Band 2, S. 1686, Band 3, S. 2328; "Houben-Weyl: Methoden der organischen Chemie", Band E V/2, 4. Auflage, Stuttgart 1985, S. 934.

Die Amide können sowohl homogen als auch auf einem inerten Träger in heterogener Form eingesetzt werden.

Die Amide können z.B. als Pulver oder Formkörper eingesetzt werden und nach der Umsetzung z.B. durch Destillation, Filtration, Sedimentation oder Zentrifugieren abgetrennt werden.

Für den Fall der Anordnung als Festbett werden die Amide vorzugsweise auf einem Träger aufgebracht und als Formkörper, z.B. als Kugeln, Zylinder, Stäbchen, Hohlzylinder, Ringe usw. eingesetzt.

Als Trägermaterial geeignet sind Aktivkohle, Kieselgel, Aluminiumoxid, Alumosilikate wie Zeolithe oder Schichtsilikate, Tonerden, Titanoxide und Zirkonoxide.

Die Amide werden bei Arbeiten mit suspendierten Katalysatoren in Rührgefäßen in Mengen von 0,1 bis 100 Gew.-%, bevorzugt von 0,5 bis 90 Gew.-% und im besonderen bevorzugt von 1,0 bis 80 Gew.-%, bezogen auf Dibenzylether, verwendet.

Im Falle einer kontinuierliche Arbeitsweise im Gegen- oder Gleichstrom oder in der Rieselphase am Festbett werden Belastungen von 0,05 g bis 5000 g Benzylchlorid pro Liter immobilisierten Amid pro Stunde, bevorzugt von 0,1 bis 4000 g/l.h und im besondere bevorzugt von 1,0 bis 3000 g/l.h verwendet.

Vorzugsweise wird das erfindungsgemäße Verfahren unter intensiver Durchmischung der Reaktionspartner durchgeführt. Eine intensive Durchmischung kann auf verschiedene, dem Fachmann bekannte Weisen, etwa durch Rühren, Düsen, Strombrecher, statische Mischer, Pumpen, turbulente Strömungen in engen Röhren und durch Ultraschall erreicht werden.

Derartige Vorrichtungen sind in Ullmann's Encyclopedia of Industrial Chemistry, 5. Auflage, Band B, Unit Operations, Abschnitte 25, 26, B4 S. 569-572, Verlag Chemie, Weinheim 1988 näher beschrieben.

Der rohe Hydroxycarbonsäurebenzylester kann beispielsweise durch Destillation oder Kristallisation weiter gereinigt werden.

Das erfindungsgemäße Verfahren kann diskontinuierlich, kontinuierlich oder teilkontinuierlich durchgeführt werden.

Eine bevorzugte Ausführungsform des erfindungsgemäßen Verfahrens ist indem man in eine Mischung oder Suspension von Amid, gegebenenfalls aufgebracht auf einem Träger, und Hydroxybenzoesäure Benzylchlorid zugibt und nach Beendigung der Reaktion das Amid oder dessen Salz durch z.B. Destillation, Filtration oder Zentrifugieren abtrennt.

Eine weitere bevorzugte Durchführungsform ist das Gleichstromverfahren, bei der Benzylchlorid und Hydroxybenzoesäure im Gleichstrom beispielsweise von oben her auf eine in einem Rohr angeordnete geträgerte Amidschüttung aufgebracht und unten am Fuß des Rohres Hydroxybenzoesäurebenzylester abgezogen werden.

In einer weiteren bevorzugten Ausführungsform des erfindungsgemäßen Verfahrens wird dieses in der Rieselphase durchgeführt und das geträgerte Amid liegt als Festbett vor. Bevorzugt befindet sich das Festbett in einem senkrecht stehenden Rohrreaktor, welcher vorzugsweise Zwischenböden zur besseren Verteilung des Flüssigkeitsstroms und zur besseren Benetzung der Schüttung enthält.

Die Aufarbeitung kann sowohl im Falle des suspendierten Katalysators als auch bei Festbettverfahrensvarianten so durchgeführt werden, dass ein Lösungsmittel, vorzugsweise Toluol, zu den Reaktionsprodukten gegeben wird. Nach Filtration kann der rohen Hydroxybenzoesäurebenzylester beispielsweise durch Destillation weiter gereinigt werden.

Die Temperatur, bei der das erfindungsgemäße Verfahren durchgeführt wird, beträgt vorzugsweise 15 bis 200, im besondere bevorzugt 25 bis 190, ganz besonders bevorzugt 30 bis 180°C.

Bei der Durchführung der Reaktion oberhalb 180°C muss entsprechend dem Dampfdruck unter erhöhtem Druck gearbeitet werden. Der benötigte Überdruck ist dann wenigstens gleich dem Dampfdruck des Reaktionsgemisches. Es kann bis etwa 50 bar betragen, vorzugsweise bis 25 bar.

Gegebenenfalls kann das erfindungsgemäße Verfahren unter einem üblichen Schutzgas wie beispielsweise Stickstoff, Helium oder Argon, durchgeführt werden.

Das erfindungsgemäße Verfahren kann durch die folgende Reaktionsgleichung erläutert werden:

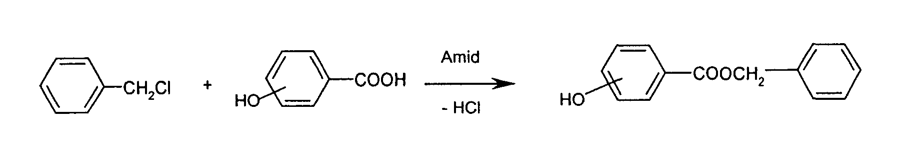

Nach dem erfindungsgemäßen Verfahren erhält man Hydroxybenzoesäurebenzylester in guten Ausbeuten bei hohem Umsatz und guter Selektivität. Das erfindungsgemäße Verfahren ist ohne hohen apparativen Aufwand einfach durchführbar.

Die Prozentangaben der nachfolgenden Beispiele beziehen sich auf das Gewicht.

### Beispiel 1

Zu einer Mischung aus 69,0 g (0,50 Mol) 2-Hydroxybenzoesäure (Salicylsäure) (Firma Merck) und 45,0 g (1,0 Mol) Formamid (Firma Aldrich) in einem Kolben mit Strombrecher und Flügelrührer wurden unter kräftigem Rühren (500 U/min) bei 150°C 63,2 g (0,50 Mol) Benzylchlorid zugegeben. Nach 1 h Reaktionszeit bei 150°C wurde rasch abgekühlt und nach lösen in Toluol die Zusammensetzung gaschromatografisch analysiert. Das Reaktionsgemisch enthält Benzylchlorid/Benzylsalicylat im Verhältnis 10,5 : 89,5.

### Beispiel 2

Beispiel 1 wurde wiederholt, aber mit 59,1 g (1,0 Mol) N-Methylformamid (Firma Aldrich). Nach 5 h Reaktionszeit enthält das Reaktionsgemisch Benzylchlorid/Benzylsalicylat im Verhältnis 26,9 : 73,1 %.

### Beispiel 3

Beispiel 1 wurde wiederholt, aber mit 73,0 g (1,0 Mol) Dimethylformamid (Firma Merck). Nach 5 h Reaktionszeit enthält das Reaktionsgemisch Benzylchlorid/Benzylsalicylat im Verhältnis 44,2 : 55,8 %.

### Beispiel 4

Beispiel 1 wurde wiederholt, aber bei 140°C. Nach 7 h Reaktionszeit enthält das Reaktionsgemisch Benzylchlorid/Benzylsalicylat im Verhältnis 50,5 : 49,5 %.

### Beispiel 5

Beispiel 1 wurde wiederholt, aber mit 59,0 g (1,0 Mol) Acetamid (Firma Acros). Nach 5 h Reaktionszeit enthält das Reaktionsgemisch Benzylchlorid/Benzylsalicylat im Verhältnis 52,1 : 47,9 %.

### Beispiel 6

Beispiel 1 wurde wiederholt, aber mit 87,0 g (1,0 Mol) Dimethylacetamid (Firma Aldrich). Nach 7 h Reaktionszeit enthält das Reaktionsgemisch Benzylchlorid/Benzylsalicylat im Verhältnis 80,7: 19,3 %.

### Beispiel 7

Beispiel 1 wurde wiederholt, aber mit 73,0 g (1,0 Mol) Benzamid (Firma Acros). Nach 5 h Reaktionszeit enthält das Reaktionsgemisch Benzylchlorid/Benzylsalicylat im Verhältnis 83,0 : 17,0 %.

### Beispiel 8

Beispiel 1 wurde wiederholt, aber mit 99,1 g (1,0 Mol) 1-Methyl-2-pyrrolidon (Firma Acros). Nach 7 h Reaktionszeit enthält das Reaktionsgemisch Benzylchlorid/Benzylsalicylat im Verhältnis 91,7 : 8,3 %.

### Beispiel 9

Beispiel 1 wurde wiederholt, aber mit 113,0 g (1,0 Mol) Caprolactam (Firma Bayer). Nach 7 h Reaktionszeit enthält das Reaktionsgemisch Benzylchlorid/Benzylsalicylat im Verhältnis 76,4 : 23,6 %.

### Beispiel 10

Beispiel 1 wurde wiederholt, aber mit 127,2 g (1,0 Mol) N-Methylcaprolactam (Firma Aldrich). Nach 7 h Reaktionszeit enthält das Reaktionsgemisch Benzylchlorid/Benzylsalicylat im Verhältnis 87,2 : 12,8 %.

### Beispiel 11

Beispiel 1 wurde wiederholt, aber mit 60,0 g (1,0 Mol) Harnstoff (Firma Grüssing). Nach 7 h Reaktionszeit enthält das Reaktionsgemisch Benzylchlorid/Benzylsalicylat im Verhältnis 87,2 : 12,8 %.

### Beispiel 12

Beispiel 1 wurde wiederholt, aber mit 30,0 g (0,5 Mol) Harnstoff (Firma Grüssing). Nach 3 h Reaktionszeit enthält das Reaktionsgemisch Benzylchlorid/Benzylsalicylat im Verhältnis 7,2 : 92,8 %.

### Beispiel 13

Beispiel 1 wurde wiederholt, aber mit 44,1 g (0,5 Mol) N,N-Dimethylhamstoff (Firma Acros). Nach 1 h Reaktionszeit enthält das Reaktionsgemisch Benzylchlorid/Benzylsalicylat im Verhältnis 40,6 : 59,4 %.

### Beispiel 14

Beispiel 1 wurde wiederholt, aber mit 58,1 g (0,5 Mol) Tetramethylhamstoff (Firma Acros). Nach 5 h Reaktionszeit enthält das Reaktionsgemisch Benzylchlorid/Benzylsalicylat im Verhältnis 44,1 : 55,9 %.

### Beispiel 15

Beispiel 3 wurde wiederholt, aber mit 69,0 g (0,5 Mol) 3-Hydroxybenzoesäure (Firma Acros). Nach 7 h Reaktionszeit enthält das Reaktionsgemisch Benzylchlorid/3-Hydroxyphenylcarbonsäurebenzylester im Verhältnis 55,3 : 44,7 %.

### Beispiel 15

Beispiel 3 wurde wiederholt, aber mit 69,0 g (0,5 Mol) 4-Hydroxybenzoesäure (Firma Acros). Nach 7 h Reaktionszeit enthält das Reaktionsgemisch Benzylchlorid/4-Hydroxyphenylcarbonsäurebenzylester im Verhältnis 54,5 : 45,5 %.

### Beispiel 16 (Aufarbeitung)

Beispiel 3 wurde wiederholt. Nach 7 h Reaktionszeit enthält das Reaktionsgemisch Benzylchlorid/Benzylsalicylat im Verhältnis 31,2 : 68,8 % und wird durch Vakuumdestillation aufgetrennt.

Man isoliert 20,3 g Benzylchlorid (30°C/2,5 mbar) und 47,8 g Benzylsalicylat (136°C/0,5 mbar), d.h. 61,8 % Benzylsalicylat bezogen auf umgesetztem Benzylchlorid.

### Beispiel 17 (Vergleichsbeispiel)

Beispiel 1 wurde wiederholt, aber ohne Amid. Nach 7 h Reaktionszeit enthält das Reaktionsgemisch Benzylchlorid/Benzylsalicylat im Verhältnis 89,3 : 10,7 %.

## Patentansprüche

1. Verfahren zur Herstellung von Hydroxybenzoesäurebenzylestern der Formel 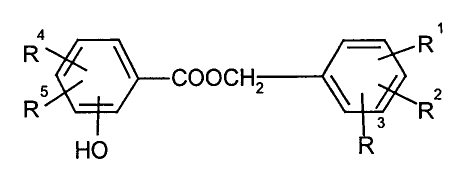 worin
R¹ bis R⁵ gleich oder verschieden sind und für Wasserstoff, C₁-C₆-Alkyl, C₁-C₆-Alkoxy-, C₁-C₆-Halogenalkyl, C₁-C₆-Halogenalkoxy-, CN, CO(C₁-C₆-Alkyl), NO₂ oder Halogen stehen,
aus Benzylchloriden, das **dadurch gekennzeichnet ist, dass** Benzylchloride der Formel 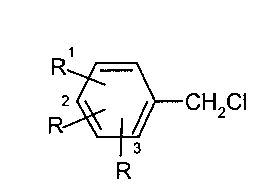 worin
R¹, R² und R³ die oben genannte Bedeutung haben,
oder Gemische aus Dibenzylethern und Benzylalkoholen der Formel 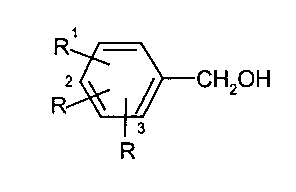 worin
R¹, R² und R³ die oben genannte Bedeutung haben,
mit Hydroxybenzoesäuren der Formel 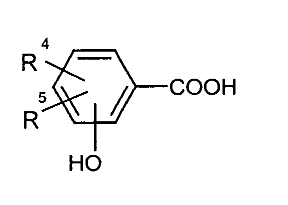 worin
R⁴ und R⁵ die oben genannte Bedeutung haben,
in Gegenwart eines oder mehrerer, bevorzugt eines Amids der Formel
R⁶CONR⁷R⁸
worin
R⁶ Wasserstoff, C₁-C₆-Alkyl, C₁-C₆-Cycloalkyl, C₁-C₆-Alkoxy-, Amino-, C₁-C₆-Alkylamino, C₁-C₆-Cycloalkylamino oder C₁-C₆-Dialkylamino bedeutet und
R⁷ und R⁸ gleich oder verschieden sind und Wasserstoff, C₁-C₆-Alkyl, C₁-C₆-Cycloalkyl, C₁-C₁₀-Aralkyl oder C₁-C₁₂-Aryl bedeuten,
oder
R⁶ und R⁷ gemeinsam eine Gruppe (CH₂)ₙ oder NR⁹(CH₂)ₙ bilden,
worin
R⁹ Wasserstoff, C₁-C₆-Alkyl, C₁-C₆-Cycloalkyl, C₁-C₁₀-Aralkyl oder C₁-C₁₂-Aryl und
n 2 bis 6 bedeutet,
umgesetzt werden.

2. Verfahren nach Anspruch 1, **dadurch gekennzeichnet dass** als Amide acyclische Amide eingesetzt werden.

3. Verfahren nach Anspruch 1, **dadurch gekennzeichnet, dass** als Amide cyclische Amide (Lactame) eingesetzt werden.

4. Verfahren nach Anspruch 1, **dadurch gekennzeichnet, dass** als Amide Harnstoffe eingesetzt werden.

5. Verfahren nach Anspruch 1, **dadurch gekennzeichnet, dass** als Amide Urethane eingesetzt werden.

6. Verfahren nach den Ansprüchen 1 bis 5, **dadurch gekennzeichnet, dass** als Amide Formamid, N-Methylformamid, Dimethylformamid, Acetamid, N-Methylacetamid, Harnstoff, Dimethylhamstoff, Tetramethylharnstoff, 2-Pyrrolidinon, N-Methyl-2-pyrrolidinon, N-Ethyl-2-pyrrolidinon, N-Cyclohexyl-2-pyrrolidinon, 2-Caprolactam, N-Methyl-2-caprolactam und N-Ethyl-2-caprolactam eingesetzt werden.

7. Verfahren nach den Ansprüchen 1 bis 6, **dadurch gekennzeichnet, dass** unsubstituiertes Benzylchlorid eingesetzt wird.

8. Verfahren nach den Ansprüchen 1 bis 7, **dadurch gekennzeichnet, dass** das Benzylchlorid ein substituiertes Benzylchlorid der Formel ist 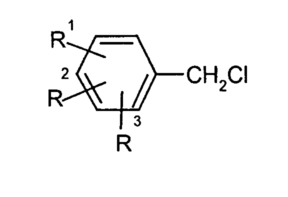 worin
R¹ bis R³ gleich oder verschieden sind und für einen oder mehrere Substituenten aus der Reihe C₁-C₆-Alkyl, C₁-C₆-Alkoxy-, C₁-C₆-Halogenalkyl, C₁-C₆-Halogenalkoxy-, CN, CO(C₁-C₆-Alkyl), NO₂ oder Halogen stehen.

9. Verfahren nach den Ansprüchen 1 bis 8, **dadurch gekennzeichnet, dass** 0,1 bis 50 Mol an Hydroxybenzoesäure, bezogen auf 1 Mol Benzylchlorid, eingesetzt werden.

10. Verfahren nach den Ansprüchen 1 bis 9, **dadurch gekennzeichnet, dass** die Umsetzung unter Entfernung von Chlorwasserstoff durch Durchleiten von Stickstoff stattfindet.

11. Verfahren nach den Ansprüchen 1 bis 10, **dadurch gekennzeichnet, dass** 0,1 bis 10 Mol Amid, bezogen auf 1 Mol Benzylchlorid eingesetzt werden.

12. Verfahren nach den Ansprüchen 1 bis 11, **dadurch gekennzeichnet, dass** die Umsetzung bei einer Temperatur im Bereich von 10 bis 200°C durchgeführt wird.
